# EUROPÄISCHE PATENTSCHRIFT

(11) **EP 0 987 242 B1**
(45) Veröffentlichungstag und Bekanntmachung des Hinweises auf die Patenterteilung: **04.12.2002**
(21) Anmeldenummer: 99115271.1
(22) Anmeldetag: 31.07.1999
(51) Int. Cl.: C07C 29/16, C07C 31/125, C07C 29/141

(54) **Verfahren zur Herstellung von höheren Oxo-Alkoholen aus Olefingemsichen**
Process for the preparation of higher oxo-alcohols from olefin mixtures
Procédé pour la préparation d'alcools oxo supérieurs à partir de mélanges d'oléfines

(30) Priorität: 16.09.1998 DE 19842371
(43) Veröffentlichungstag der Anmeldung: 22.03.2000
(73) Patentinhaber: Oxeno Olefinchemie GmbH, 45764 Marl (DE)
(72) Erfinder: Kaizik, Alfred, Dr., 45772 Marl (DE); Scholz, Bernhard, Dr., 45768 Marl (DE); Tötsch, Walter, Dr., 45770 Marl (DE); Trocha, Martin, Dr., 45136 Essen (DE); Büschken, Wilfried, Dr., 45721 Haltern (DE); Nierlich, Franz, Dr., 45768 Marl (DE)

(56) Entgegenhaltungen:
- EP-A- 0 183 545
- EP-A- 0 326 674
- DE-A- 1 935 900
- DE-A- 3 232 557
- FR-A- 2 322 119
- PATENT ABSTRACTS OF JAPAN vol. 013, no. 423 (C-638), 20. September 1989 (1989-09-20) & JP 01 160928 A (MITSUBISHI KASEI CORP), 23. Juni 1989 (1989-06-23)

## Beschreibung

Die Erfindung betrifft ein Verfahren zur Herstellung höherer Oxo-Alkohole durch Hydroformylierung von Olefingemischen, das eine selektive Hydrierung der Hydroformylierungsgemische sowie eine Rückführung der nicht umgesetzten Olefine einschließt.

### Stand der Technik

Höhere Alkohole, insbesondere solche mit 6 bis 25 C-Atomen, können bekanntlich durch katalytische Hydroformylierung (oder Oxo-Reaktion) der um ein C-Atom ärmeren Olefine und anschließende katalytische Hydrierung der Aldehyd- und Alkohol-haltigen Reaktionsgemische hergestellt werden. Sie werden vorwiegend als Edukte für die Herstellung von Weichmachern oder Detergentien verwendet.

Die Art des Katalysatorsystems und die optimalen Reaktionsbedingungen für die Hydroformylierung sind von der Reaktivität des eingesetzten Olefins abhängig. Die Abhängigkeit der Reaktivität der Olefine von ihrer Struktur ist z.B. von J.Falbe. New Syntheses with Carbon Monoxide, Springer-Verlag, Berlin, Heidelberg, New York, 1980, Seite 95 ff., beschrieben. Die unterschiedliche Reaktivität speziell der isomeren Octene ist ebenfalls bekannt (B.L.Haymore, A. van Hasselt, R.Beck, Annals of the New York Acad.Sci.. **415** (1983). Seiten 159-175).

Technische Olefingemische, die als Edukte für die Oxo-Synthese verwendet werden, enthalten Olefinisomere der verschiedensten Strukturen mit unterschiedlichen Verzweigungsgraden, unterschiedlicher Lage der Doppelbindung im Molekül und gegebenenfalls auch unterschiedlichen C-Zahlen. Dies gilt besonders für Olefingemische, die durch Di-. Tri- oder weitergehende Oligomerisierung von C₂-C₅-Olefinen oder anderen leicht zugänglichen höheren Olefinen bzw. durch Cooligomerisierung der genannten Olefine entstanden sind. Als Beispiele für typische isomere Olefingemische, die durch Rhodium-katalysierte oder vorzugsweise durch Kobalt-katalysierte Hydroformylierung zu den entsprechenden Aldehyd- und Alkohol-Gemischen umgesetzt werden können, seien Tri- und Tetrapropene sowie Di-. Tri- und Tetrabutene genannt.

Die Geschwindigkeit der Hydroformylierungsreaktion nimmt mit steigender C-Zahl sowie mit dem Verzweigungsgrad ab. Die Reaktionsgeschwindigkeit von linearen Olefinen kann um den Faktor 5 bis 10 größer sein als die der verzweigten Isomeren. Auch die Lage der Doppelbindung im Olefinmolekül beeinflußt die Reaktivität. Olefine mit endstardiger Doppelbindung reagieren deutlich schneller als Isomere mit der Doppelbindung im Inneren des Moleküls. Wegen der unterschiedlichen Reaktivität der Olefinisomeren bedarf es relativ langer Reaktionszeiten, wenn man einen möglichst weitgehenden Umsatz der Olefine erreichen will. Dadurch wird jedoch die Produktausbeute infolge unerwünschter Neben- und Folgereaktionen vermindert. Dasselbe tritt ein, wenn man die Reaktionszeiten durch höhere Reaktionstemperaturen abzukürzen trachtet. Vor allem wegen der unterschiedlichen Reaktivität der Isomeren ist es schwierig, bei der Hydroformylierung von Olefingemischen hohe Umsätze und gleichzeitig hohe Selektivitäten zu erzielen.

Die Herstellung von Alkoholen durch Hydroformylierung von Olefinen und Hydrierung der so erhaltenen Aldehyde ist bekannt; es wurden daher bereits viele Vorschläge zur Erhöhung der Selektivität dieser Reaktionen gemacht. So beschreibt DE 32 32 557 ein zweistufiges Hydroformylierungsverfahren, bei dem die zweite Hydroformylierungsstufe gleichzeitig als Hydrierreaktion ausgeführt wird. Die Selektivitäten, die insbesondere mit der zweiten Reaktionsstufe erreicht werden können, sind verbesserungsfähig.

In DE 25 38 037 wird ebenfalls ein zweistufiges Verfahren zur Hydroformylierung von Olefinen beschrieben, wobei die Reaktorausträge der Hydroformylierungsstufen einer Totalhydrierung unterzogen werden. Nicht umgesetzte. Olefine werden auf diese Weise zu den entsprechenden aliphatischen Verbindungen hydriert und gehen dem Prozess verloren. Auch in JP 11 60 928 ist eine Hydroformylierungsreaktion von Olefinen, gefolgt von einer Totalhydrierung des Reaktoraustrags beschrieben.

Eine selektive Hydrierung von Produktströmen, die Aldehyde und nicht umgesetzte Olefine aus Hydroformylierungsreaktionen enthalten ist in DE 19 35 900 offenbart.

Die genannten Verfahren sind hinsichtlich Umsatz und Selektivität noch verbesserungswürdig.

### Kurzbeschreibung der Erfindung

Eine Aufgabe der Erfindung besteht darin, ein Verfahren zur Herstellung von höheren Oxo-Alkoholen aus den entsprechenden Olefingemischen bereitzustellen, das hohe Umsätze mit hohen Selektivitäten verbindet und sich zudem durch hohe Raum-Zeit-Ausbeuten auszeichnet.

Gegenstand der Erfindung ist dementsprechend ein Verfahren zur Herstellung von höheren Oxo-Alkoholen aus Gemischen isomerer Olefine mit 5 bis 24 Kohlenstoffatomen durch Hydroformylierung in Gegenwart eines Katalysators bei erhöhter Temperatur und unter erhöhtem Druck, bei dem man die Hydroformylierung in einer Stufe durchführt, den Umsatz der Olefine für einen Durchgang auf 40 bis 90 % begrenzt, das Reaktionsgemisch, zweckmäßig nach Katalysatorabtrennung, selektiv hydriert, das Hydrierungsgemisch durch Destillation auftrennt und die Olefinfraktion in die Hydroformylierung zurückführt.

### Beschreibung der Erfindung

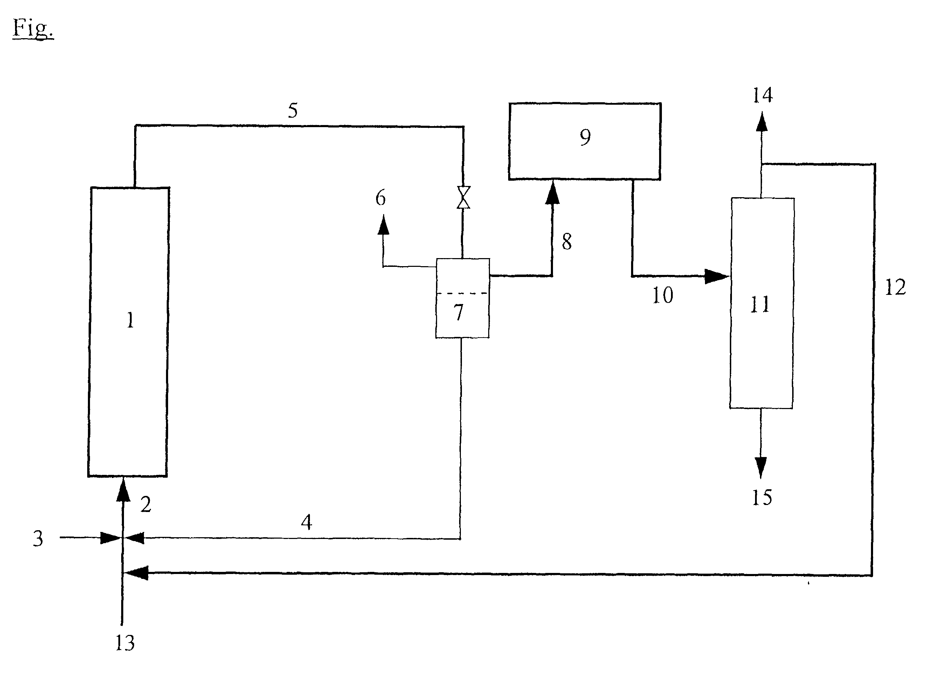

Das erfindungsgemäße Verfahren kann diskontinuierlich durchgeführt werden, vorteilhaft ist jedoch eine kontinuierliche Arbeitsweise. In der Figur ist das Blockschema einer Anlage dargestellt, in der das Verfahren kontinuierlich durchgeführt werden kann. In den Reaktor 1 werden das Olefingemisch 2. Synthesegas (Kohlenmonoxid und Wasserstoff) 3 sowie Katalysator 4 eingeführt. Das Hydroformylierungsgemisch 5 wird entspannt, das Entspannungsgas 6 (nicht verbrauchtes Synthesegas) wird abgezogen und das entspannte Hydroformylierungsgemisch in der Katalysatorabtrennung 7 vom Katalysator 4 befreit, der, gegebenenfalls nach Ergänzung durch frischen Katalysator, in den Reaktor 1 zurückgeführt wird. Das vom Katalysator befreite Hydroformylierungsgemisch 8 wird in die Hydrierung 9 geleitet, in der die Aldehyde sowie als Nebenprodukte enthaltene Acetale der Aldehyde und Ester der Alkohole, insbesondere deren Formiate, zu den Alkoholen hydriert werden. Aus dem Hydrierungsgemisch 10 werden in der Destillation 11 die Leichtsieder 12 abgetrennt. die weit überwiegend aus nicht umgesetzten isomeren Olefinen bestehen und zusammen mit frischen Olefinen 13 als Olefingemisch 2 in den Reaktor 1 geführt werden. Ein Teil der Leichtsieder kann als restliche Leichtsieder 14 aus dem Olefinkreislauf abgezogen werden. Das rohe Alkoholgemisch 15 wird in einer weiteren, nicht dargestellten Destillation auf reinen Alkohol aufgearbeitet.

### Hydroformylierung

Die Edukte für die Hydroformylierung sind Gemische von Monoolefinen mit 5 bis 24 Kohlenstoffatomen und end- oder mittelständiger C-C-Doppelbindung, wie 1- oder 2-Penten. 2-Methyl-1-buten, 1-, 2- oder 3-Hexen, das bei der Dimerisierung von Propen anfallende isomere C₆-Oleflngemlsch (Dipropen), 1-Hepten, 2- oder 3-Methyl-1-hexen, 1-Octen, das bei der Dimerisierung von Butenen anfallende isomere C₈-Olefingemisch (Dibuten), 1-Nonen, 2-, 3- oder 4-Methyl-1-octen, das bei der Trimerisierung von Propen anfallende isomere C₉-Olefingemisch (Tripropen), 1-, 2- oder 3-Decen, 2-Ethyl-1-octen, 1-Dodecen, das bei der Tetramerisierung von Propen oder der Trimerisierung von Butenen anfallende isomere C₁₂-Olefingemisch (Tetrapropen oder Tributen), 1-Tetradecen, 1- oder 2-Hexadecen, bei der Tetramerisierung von Butenen anfallende C₁₆-Olefingemische (Tetrabuten) sowie durch Cooligomerisierung von Olefinen mit unterschiedlichen C-Zahlen (bevorzugt 2 bis 4) hergestellte Olefingemische, gegebenenfalls nach destillativer Trennung in Fraktionen mit gleicher oder ähnlicher C-Zahl. Bevorzugte Edukte sind C₈-, C₉-, C₁₂- oder C₁₆-Olefingemische.

Die Erfindung liegt nicht in der Art oder den Bedingungen der Hydroformylierung. Die Olefine werden vielmehr in an sich bekannter Weise hydroformyliert. Man arbeitet also mit Rhodium- oder vorzugsweise mit Kobaltkatalysatoren sowie mit oder ohne Komplex-stabilisierende Zusätze, wie organische Phosphine oder Phosphite. Die Temperaturen und die Drücke können, je nach Katalysator und Olefingemisch, in weiten Grenzen variieren. Eine Beschreibung der Hydroformylierung von Olefinen findet sich z.B. bei J.Falbe, New Syntheses with Carbon Monoxide, Springer-Verlag, Heidelberg-New York, 1980, Seiten 99ff. sowie bei Kirk-Othmer, Encyclopedia of Chemical Technology, Band 17, 4. Auflage, John Wiley & Sons. Seiten 902-919 (1996).

Ein wesentliches Merkmal der Erfindung besteht darin, daß man den Umsatzgrad für einen Durchgang auf 40 bis 90% begrenzt. Vorteilhaft setzt man die Olefine zu 65 bis 80% um. Die bevorzugten Edukte sind C₈-, C₉-, C₁₂- oder C₁₆-Olefingemische, die aus einer ganzen Anzahl verschiedener Isomerer bestehen. Wie zuvor erwähnt, reagieren geradkettige Olefine mit endständigen olefinischen Doppelbindungen am bereitwiligsten. Die Reaktionsfähigkeit nimmt umso mehr ab, je stärker das Molekül verzweigt ist und/oder je weiter die Doppelbindung im Inneren des Moleküls liegt. Die erfindungsgemäße Umsatzbegrenzung bewirkt, daß die reaktionsfähigen Olefine bevorzugt abreagieren, die weniger reaktionsfähigen im Reaktionsgemisch verbleiben und nach der selektiven Hydrierung des Reaktionsgemisches in die Hydroformylierung zurückgeführt werden. Durch die Umsatzbegrenzung wird die Selektivität der Hydroformylierung erhöht. Durch Olefinrückführung wird die Gesamtverweilzeit für die reaktionsträgeren Olefine verlängert. Als Folge der Olefin-Rückführung werden höhere Olefin-Gesamtumsätze bei geringerer Nebenproduktbildung und somit höhere Aldehyd-Ausbeuten und, nach Hydrierung, höhere Alkohol-Ausbeuten erreicht. Darüberhinaus erleichtert die kleinere Menge an Nebenprodukten die Aufarbeitung der Reaktionsgemische. Im Vergleich zum einstufigen Verfahren ohne Olefinrückführung erhöht das erfindungsgemäße Verfahren aufgrund der Selektivhydrierung und der Olefinrückführung die Wirtschaftlichkeit der Herstellung von Oxo-Alkoholen.

Der Umsatzgrad der Olefine wird auf den gewünschten Wert begrenzt. indem man die Reaktionsbedingungen der Hydroformylierung zweckentsprechend verändert. Durch Wahl von niedrigeren Reaktionstemperaturen und/oder Katalysatorkonzentrationen sowie kürzeren Reaktionszeiten kann der Olefin-Umsatz gesenkt werden. Der Umsatzgrad für einen Durchgang des Olefingemisches 2 (= frisches Olefingemisch 13 + zurückgeführte Leichtsieder 12) wird auf Basis der Menge und der Zusammensetzung des Olefingemisches 2 sowie der Menge und der Zusammensetzung der zurückgeführten Leichtsieder 12 zuzüglich der abgezogenen Leichtsieder 14 bestimmt Der Gesamtumsatzgrad wird auf Basis der Menge und der Zusammensetzung des frischen Olefingemisches 13 sowie der mit den restlichen Leichtsiedern 14 ausgeschleusten Olefinmengen bestimmt. Für die Bestimmung der Olefingehalte in den verschiedenen Strömen kann die gaschromatographische Analyse angewandt werden.

### Katalysatorabtrennung

Die Reaktionsgemische der Hydroformylierung werden zweckmäßig zunächst vom Katalysator befreit, wiederum in an sich bekannter Weise. Wenn ein Kobaltkatalysator verwendet wurde, kann dies durch Druckentlastung, Abtrennung der wäßrigen Katalysatorphase, Oxidation der im Hydroformylierungsgemisch verbliebenen Kobaltcarbonylverbindungen mit Luft oder Sauerstoff und Auswaschen der entstandenen Kobaltverbindungen mit Wasser oder wäßriger Säure geschehen. Entkobaltungsverfahren sind gut bekannt, siehe z.B. J.Falbe, a.a.O., 164, 165 (BASF-Process). Kirk-Othmer, a.a.O., sowie EP-0 850 905 A1. Wenn eine Rhodiumverbindung als Hydroformylierungskatalysator diente, kann man sie mittels Dünnschichtverdampfung als Destillationsrückstand vom Hydroformylierungsgemisch abtrennen.

Die von Katalysator befreiten Reaktionsgemische der Hydroformylierung enthalten, je nach dem Umsatzgrad, im allgemeinen 3-40 Gew. -%, meistens 5 bis 30 Gew.-% Leichtsieder mit niedrigerem Siedepunkt als die Aldehyde, hauptsächlich Olefine, daneben die entsprechenden gesättigten Kohlenwasserstoffe sowie Wasser und gegebenenfalls Methanol, weiterhin 30-90 Gew.-% Aldehyde, 5-60 Gew.-% Alkohole, bis zu 10 Gew.-% Formiate dieser Alkohole und 5-15 Gew.-% Hochsieder mit höherem Siedepunkt als die Alkohole. Es sei jedoch betont, daß das erfindungsgemäße Verfahren auch mit Hydroformylierungsgemischen ausgeführt werden kann, deren Zusammensetzung in dieser und/oder jener Beziehung nicht diesen Angaben entspricht.

### Selektive Hydrierung

Die selektive Hydrierung der zweckmäßig vom Hydroformylierungskatalysator befreiten Hydroformylierungsgemische ist ein weiteres wesentliches Merkmal des erfindungsgemäßen Verfahrens. Dabei werden die Aldehyde und bestimmte Begleitstoffe, darunter Acetale der Aldehyde sowie Ester der Alkohole und von diesen insbesondere die Formiate, zu den gewünschten Alkoholen hydriert. Da der Umsatz in der Hydroformylierungsstufe begrenzt wird, ist es für die Wirtschaftlichkeit des Verfahrens entscheidend, daß bei der Hydrierung die nicht umgesetzten Olefine nicht oder praktisch nicht hydriert werden, so daß sie aus dem Hydrierungsgemisch abgetrennt und in die Hydroformylierung zurückgeführt werden können.

Eine selektive Hydrierung von Hydroformylierungsgemischen ist Gegenstand der gleichzeitig anhängigen Patentanmeldung 198 42 370.5 (O.Z. 5356). Die Reaktionsgemische der Hydroformylierung werden danach mittels Wasserstoff bei erhöhter Temperatur und unter erhöhtem Druck an einem Trägerkatalysator hydriert, der als aktive Komponenten Kupfer, Nickel und Chrom enthält.

Bevorzugte Katalysatoren dieser Art sind Trägerkatalysatoren, die als aktive Komponenten Kupfer und Nickel in Konzentrationen von jeweils 0.3 bis 15 Gew. -%. Chrom in einer Konzentration von 0.05 bis 3.5 Gew.-% sowie eine Alkalimetallkomponente in einer Konzentration von 0.01 bis 1.6 Gew.-%, vorteilhaft von 0.02-1.2 Gew.-% enthalten, jeweils bezogen auf den Trägerkatalysator. Ein anderer vorteilhafter Trägerkatalysator enthält Kupfer, Nickel und Chrom in den angegebenen Mengen, aber keine Alkalimetallkomponente. Geeignete Trägerstoffe sind insbesondere Siliciumdioxid und Aluminiumoxid. Die Mengenangaben beziehen sich auf den wie folgt beschrieben hergestellten, noch nicht reduzierten Katalysator.

Bei der Hydrierung werden die Aldehyde in den Hydroformylierungsgemischen bei Umsätzen über 98% in einer Selektivität von über 99% in nur einer Hydrierstufe zu den entsprechenden Alkoholen hydriert. Die Ester und die Acetale werden ebenfalls in die gewünschten Alkohole umgewandelt. Die im Gemisch enthaltenen Ausgangsolefine bleiben überraschenderweise weit überwiegend unverändert, obwohl gerade die bevorzugten Trägerkatalysatoren unter vergleichbaren Bedingungen auch die olefinische Doppelbindung im 2-Ethylhex-2-enal praktisch quantitativ hydrieren (EP 0 326 674 A2). Die Hydrierung kann im Niederdruckbereich von unter 30 bar und mit hohen Raum-Zeit-Ausbeuten durchgeführt werden.

Die genannten Katalysatorkomponenten können homogen in den Poren eines Trägermaterials verteilt oder in dessen Randzonen angereichert sein. Im ersteren Fall setzt man eine wässerige Lösung an, die die Komponenten in Form von Metallsalzen als Katalysatorvorläufer enthält und deren Volumen zweckmäßig ungefähr dem 0,8-fachen des Porenvolumens des Trägermaterials entspricht. Als Kupfer-, Nickel- und Chromsalze verwendet man vorteilhaft solche, die beim Erhitzen in Oxide übergehen, wie Nitrate und Acetate. Wenn der Katalysator eine Alkalimetallkomponente enthalten soll, kann diese zusammen mit Chrom in Form von Alkalichromat oder -dichromat, insbesondere als Natriumchromat oder -dichromat eingebracht werden. Die Konzentration der Metallsalze in der Lösung hängt von der gewünschten Konzentration der jeweiligen Komponente im fertigen Katalysator ab. Die Metallsalzlösung wird dann auf das in einer Dragiertrommel befindliche, nicht vorerhitzte Trägermaterial aufgesprüht und dringt in dessen Poren ein. Danach wird der Katalysator getrocknet.

Ist ein Katalysator mit Komponenten erwünscht, die in den Randzonen eines porösen oder eines mehr oder minder porenfreien Trägermaterials angereichert sind, so kann man die Metallsalzlösung auf ein vorerhitzte Trägermaterial aufsprühen und das Trägermaterial während des Aufsprühens weiter erhitzen, so daß das Wasser verdampft und die Katalysatorkomponenten im wesentlichen auf der Oberfläche des Trägermaterials fixiert werden.

Nach dem Aufbringen der Katalysatorkomponenten werden die Katalysatoren beider genannten Typen calciniert, d.h. je nach verwendetem Katalysator-Vorläufer auf Temperaturen von 200-400°C erhitzt, wobei die Katalysatorvorläufer in die Oxide umgewandelt werden. Danach wird der Katalysator mit Wasserstoff reduziert. Die Reduktion kann gleich nach der Herstellung des Katalysators oder zweckmäßig erst in dem Hydrierungsreaktor erfolgen.

Die Katalysatoren werden vorteilhaft in einer Form eingesetzt, in der sie einen geringen Strömungswiderstand bieten, z.B. in Form von Granalien, Pellets oder Formkörpern, wie Tabletten, Zylindern, Strangextrudaten oder Ringen. Sie werden zweckmäßig vor ihrem Einsatz durch Erhitzen im Wasserstoffstrom. z.B. auf die genannten Hydriertemperaturen, 150 bis 250°C, aktiviert, sofern sie nicht im Reaktor reduziert wurden.

Die Hydrierung kann kontinuierlich oder diskontinuierlich und sowohl in der Gasphase als auch in flüssiger Phase durchgeführt werden. Die Hydrierung in flüssiger Phase wird bevorzugt, weil das Gasphasenverfahren wegen der notwendigen Kreisführung großer Gasvolumina einen höheren Energieaufwand erfordert. Hinzu kommt, daß die Verdampfung der Aldehyde mit steigender C-Zahl mehr und mehr Energie erfordert und zudem die Eduktbeladung im Reaktionsgas abnimmt, so daß ein Gasphasenverfahren bei Aldehyden mit einer C-Zahl größer als etwa 8 das Gesamtverfahren kaum noch wirtschaftlich durchführbar macht.

Für die Flüssigphasenhydrierung können unterschiedliche Verfahrensvarianten gewählt werden. Sie kann adiabatisch oder praktisch isotherm, d.h. mit einem Temperaturanstieg von <10°C, ein- oder zweistufig durchgeführt werden. Im letzteren Fall kann man beide Reaktoren, zweckmäßig Rohrreaktoren, adiabatisch oder praktisch isotherm oder den einen adiabatisch und den anderen praktisch isotherm betreiben. Weiterhin ist es möglich, die Hydroformylierungsgemische im geraden Durchgang oder mit Produktrückführung zu hydrieren. Die Reaktoren können als Gleichstromreaktoren mit Rieselbett (trickle flow) oder bevorzugt mit hohen Flüssigkeitsbelastungen (pulse flow) betrieben werden. Im Interesse einer hohen Raum-Zeit-Ausbeute werden die Reaktoren vorzugsweise mit hohen Flüssigkeitsbelastungen von 5-100 m³, insbesondere von 15-50 m³ pro m² Querschnitt des leeren Reaktors und Stunde betrieben. Wird ein Reaktor isotherm und im geraden Durchgang betrieben, so kann die spezifische Katalysatorbelastung (LHSV) Werte zwischen 0.1 und 10 h⁻¹, vorzugsweise zwischen 0.5 und 5 h⁻¹ annehmen.

Die Flüssigphasenhydrierung wird im allgemeinen unter einem Gesamtdruck von 5 bis 30 bar, insbesondere zwischen 15 und 25 bar durchgeführt. Die Hydrierung in der Gasphase kann auch bei niedrigeren Drükken durchgeführt werden, mit entsprechend größeren Gasvolumina. Die Reaktionstemperaturen liegen bei Hydrierungen in flüssiger oder gasförmiger Phase in der Regel zwischen 120 und 220°C, insbesondere zwischen 140 und 180°C.

### Auftrennung des Hydrierungsgemisches durch Destillation

Nach der Hydrierung werden die Reaktionsgemische in an sich bekannter Weise durch Destillation aufgearbeitet. Dies geschieht zweckmäßig unter vermindertem Druck, z.B. bei einem absoluten Druck von 400 bis 900 mbar. Dabei werden die Olefine als weit überwiegender Bestandteil der Leichtsiederfraktion zurückgewonnen. Zweckmäßig wird der überwiegende Teil der Leichtsiederfraktion. in der Regel 60 bis 98 %, in die Hydroformylierung zurückgeführt. Den restliche Anteil der Leichtsiederfraktion, also 2 bis 40%, kann man aus dem Olefinkreislauf ausschleusen, damit die Konzentration der inerten, durch Hydrierung der Olefine in der Hydroformulierungsstufe entstandenen gesättigten Kohlenwasserstoffe nicht 70% übersteigt, vorzugsweise unter 60% gehalten wird.

## Patentansprüche

1. Verfahren zur Herstellung höherer Oxo-Alkohole aus Gemischen isomerer Olefine mit 5 bis 24 Kohlenstoffatomen durch Hydroformylierung in Gegenwart eines Katalysators bei erhöhter Temperatur und unter erhöhtem Druck, **dadurch gekennzeichnet, daß** man die Hydroformylierung in einer Stufe durchführt, den Umsatz der Olefine für einen Durchgang auf 40 bis 90% begrenzt, das Reaktionsgemisch, zweckmäßig nach Katalysatorabtrennung, selektiv bei erhöhter Temperatur und unter erhöhtem Druck an einem Trägerkatalysator hydriert, der als aktive Komponenten Kupfer, Nickel und Chrom enthält, das Hydriergemisch durch Destillation auftrennt und die Olefinfraktion in die Hydroformylierung zurückführt.

2. Verfahren nach Anspruch 1, **dadurch gekennzeichnet**, man C₈-, C₉-, C₁₂- oder C₁₆-Olefingemische als Edukte für die Hydroformylierung einsetzt.

3. Verfahren nach Anspruch 2, **dadurch gekennzeichnet, daß** man einen Trägerkatalysator verwendet, der als aktive Komponenten Kupfer und Nickel in Konzentrationen von jeweils 0.3 bis 15 Gew.-%, Chrom in einer Konzentration von 0.05 bis 3.5 Gew.-% und eine Alkalimetallkomponente in einer Konzentration von 0.01 bis 1.6 Gew.-%, jeweils bezogen auf den Trägerkatalysator, enthält.

4. Verfahren nach Anspruch 3, **dadurch gekennzeichnet, daß** die Konzentration der Alkalimetallkomponente 0,2-1,2 Gew. -% beträgt.

5. Verfahren nach Anspruch 3, **dadurch gekennzeichnet, daß** der Trägerkatalysator keine Alkalimetallkomponente enthält.

6. Verfahren nach einem der Ansprüche 1 bis 5, **dadurch gekennzeichnet, daß** das Trägermaterial des Katalysators Siliciumdioxid oder Aluminumoxid ist.

7. Verfahren nach einem der Ansprüche 1 bis 6, **dadurch gekennzeichnet, daß** die aktiven Katalysatorkomponenten homogen in den Poren des Trägerstoffs verteilt sind.

8. Verfahren nach einem der Ansprüche 1 bis 7, **dadurch gekennzeichnet daß** die genannten Katalysatorkomponenten homogen in den Randzonen des Trägerstoffs angereichert sind.

9. Verfahren nach einem der Ansprüche 1 bis 8, **dadurch gekennzeichnet, daß** man die Hydrierung kontinuierlich oder diskontinuierlich in flüssiger Phase durchführt.

10. Verfahren nach einem der Ansprüche 1 bis 9, **dadurch gekennzeichnet, daß** man die Hydrierung in flüssiger Phase unter einem Gesamtdruck von 5 bis 30 bar durchführt.

11. Verfahren nach Anspruch 10, **dadurch gekennzeichnet, daß** der Gesamtdruck 15 bis 25 bar beträgt.

12. Verfahren nach einem der Ansprüche 1 bis 11, **dadurch gekennzeichnet, daß** man die Hydrierung bei 120 bis 220°C durchführt.

13. Verfahren nach Anspruch 12, **dadurch gekennzeichnet, daß** die Temperatur 140 bis 180°C beträgt.

14. Verfahren nach einem der Ansprüche 1 bis 13, **dadurch gekennzeichnet, daß** man die Hydrierung in flüssiger Phase und mit Flüssigkeitsbelastungen von 5-100 m³ pro m² Querschnitt des leeren Reaktors und Stunde durchführt.

15. Verfahren nach Anspruch 14, **dadurch gekennzeichnet, daß** die Flüssigkeitsbelastung 15-50 m³ pro m² Querschnitt des leeren Reaktors und Stunde beträgt.

16. Verfahren nach einem der Ansprüche 1 bis 15, **dadurch gekennzeichnet, daß** man das Hydrierungsgemisch durch Destillation trennt und die Olefine in die Hydroformylierung zurückführt.

17. Verfahren nach Anspruch 16, **dadurch gekennzeichnet, daß** man 2 bis 40 % der Leichtsieder aus dem Olefinkreislauf ausschleust.

## Claims

1. A process for preparing higher oxo alcohols from mixtures of isomeric olefins having from 5 to 24 carbon atoms by hydroformylation in the presence of a catalyst at elevated temperature and at elevated pressure, **characterized in that** the hydroformylation is carried out in one stage, the olefin conversion rate for one passage is restricted to from 40 to 90%, the reaction mixture, expediently after catalyst removal, is selectively hydrogenated at elevated temperature and at elevated pressure on a supported catalyst which comprises, as active components, copper, nickel and chromium, the hydrogenation mixture is separated by distillation and the olefin fraction is recirculated to the hydroformylation.

2. A process according to claim 1, **characterized in that** use is made of C₈, C₉, C₁₂ or C₁₆ olefin mixtures as the starting materials for the hydroformylation.

3. A process according to claim 2, **characterized in that** use is made of a supported catalyst which comprises, as active components, copper and nickel at concentrations in each case of from 0.3 to 15% by weight, chromium at a concentration of from 0.05 to 3.5% by weight and an alkali metal component at a concentration of from 0.01 to 1.6% by weight, in each case based on the supported catalyst.

4. A process according to claim 3, **characterized in that** the concentration of the alkali metal component is from 0.2 to 1.2% by weight.

5. A process according to claim 3, **characterized in that** the supported catalyst does not contain an alkali metal component.

6. A process according to any one of claims 1 to 5, **characterized in that** the catalyst support material is silicon dioxide or aluminium oxide.

7. A process according to any one of claims 1 to 6, **characterized in that** the active catalyst components are homogeneously distributed in the pores of the support material.

8. A process according to any one of claims 1 to 7, **characterized in that** the said catalyst components are homogeneously enriched in the edge zones of the support material.

9. A process according to any one of claims 1 to 8, **characterized in that** the hydrogenation is carried out continuously or batchwise in the liquid phase.

10. A process according to any one of claims 1 to 9, **characterized in that** the hydrogenation is carried out in the liquid phase at an overall pressure of from 5 to 30 bar.

11. A process according to claim 10, **characterized in that** the overall pressure is from 15 to 25 bar.

12. A process according to any one of claims 1 to 11, **characterized in that** the hydrogenation is carried out at from 120 to 220°C.

13. A process according to claim 12, **characterized in that** the temperature is from 140 to 180°C.

14. A process according to any one of claims 1 to 13, **characterized in that** the hydrogenation is carried out in the liquid phase and at liquid loadings of 5-100 m³ per m² of cross section of the empty reactor and hour.

15. A process according to claim 14, **characterized in that** the liquid loading is 15-50 m³ per m² of cross section of the empty reactor and hour.

16. A process according to any one of claims 1 to 15, **characterized in that** the hydrogenation mixture is separated by distillation and the olefins are recirculated to the hydroformylation.

17. A process according to claim 16, **characterized in that** from 2 to 40% of the low-boilers are discharged from the olefin circuit.

## Revendications

1. Procédé de préparation d'oxoalcools supérieurs à partir de mélanges d'oléfines isomères ayant de 5 à 24 atomes de carbone par hydroformylation en présence d'un catalyseur, à température accrue et sous pression accrue,
**caractérisé en ce qu'**
on effectue l'hydroformylation en une seule étape, **en ce qu'**on limite la conversion des oléfines pour une opération de 40 à 90 %, et **en ce qu'**on hydrogène le mélange réactionnel d'une manière appropriée après séparation des catalyseurs, d'une manière sélective à température accrue et sous pression accrue sur un catalyseur sur support qui contient comme composants actifs du cuivre, du nickel, et du chrome, on sépare le mélange d'hydrogénation par distillation et on recycle la fraction d'oléfines dans l'hydroformylation.

2. Procédé selon la revendication 1,
**caractérisé en ce qu'**
on met en oeuvre comme éduits pour l'hydroformylation des mélanges d'oléfines en C₈, C₉, C₁₂ ou C₁₆.

3. Procédé selon la revendication 2,
**caractérisé en ce qu'**
on utilise un catalyseur sur support qui contient comme composants actifs du cuivre et du nickel à des concentrations de respectivement 0,3 à 15 % en poids, de chrome à une concentration de 0,05 à 3,5 % en poids, et un composant de métal alcalin à une concentration de 0,01 à 1,6 % en poids, respectivement rapporté au catalyseur sur support.

4. Procédé selon la revendication 3,
**caractérisé en ce que**
la concentration du composant de métal alcalin s'élève à 0,2 à 1,2 % en poids.

5. Procédé selon la revendication 3,
**caractérisé en ce que**
le catalyseur sur support ne contient pas de composant de métal alcalin.

6. Procédé selon l'une des revendications 1 à 5,
**caractérisé en ce que**
le matériau de support du catalyseur est du dioxyde de silicium ou de l'oxyde d'aluminium.

7. Procédé selon l'une des revendications 1 à 6,
**caractérisé en ce que**
les composants actifs du catalyseur sont répartis d'une manière homogène dans les pores de la substance du support.

8. Procédé selon l'une des revendications 1 à 7,
**caractérisé en ce que**
les composants cités de catalyseur sont enrichis d'une manière homogène dans les zones de bordure de la substance de support.

9. Procédé selon l'une des revendications 1 à 8,
**caractérisé en ce qu'**
on effectue l'hydrogénation en continu ou d'une manière discontinue en phase liquide.

10. Procédé selon l'une des revendications 1 à 9,
**caractérisé en ce qu'**
on effectue l'hydrogénation en phase liquide sous une pression totale de 5 à 30 bars.

11. Procédé selon la revendication 10,
**caractérisé en ce que**
la pression totale est de 15 à 25 bars.

12. Procédé selon l'une des revendications 1 à 11,
**caractérisé en ce qu'**
on effectue l'hydrogénation à 120 à 220°C.

13. Procédé selon la revendication 12,
**caractérisé en ce que**
la température est de 140 à 180°C.

14. Procédé selon l'une des revendications 1 à 13,
**caractérisé en ce qu'**
on effectue l'hydrogénation en phase liquide, et avec des contraintes de liquide de 5 à 100 m³ par m² de section du réacteur vide et par heure.

15. Procédé selon la revendication 14,
**caractérisé en ce que**
la contrainte de liquide s'élève à 15 à 50 m³ par m² de section du réacteur vide et par heure.

16. Procédé selon l'une des revendications 1 à 15,
**caractérisé en ce qu'**
on sépare le mélange d'hydrogénation par distillation et on recycle les oléfines dans l'étape d'hydroformylation.

17. Procédé selon la revendication 16,
**caractérisé en ce qu'**
on évacue du circuit d'oléfine de 2 à 40 % des fractions à bas point d'ébullition.
